# EUROPEAN PATENT APPLICATION

(11) **EP 4 707 379 A1**
(43) Date of publication of application: **11.03.2026**
(21) Application number: 24382951.2
(22) Date of filing: 04.09.2024
(51) Int. Cl.: C12N 1/20, A01N 63/20

(54) **BIOINOCULANT FOR THE PROMOTION OF GROWTH OF A MARINE PHANEROGAM**

(71) Applicant: Universidad de Las Palmas de Gran Canaria, 35001 Las Palmas de Gran Canaria (ES)
(72) Inventor: GARCÍA JIMÉNEZ, Pilar, 35017 Las Palmas de Gran Canaria (ES); ROBAINA ROMERO, Rafael, 35017 Las Palmas de Gran Canaria (ES); CARRASCO ACOSTA, Marina, 35017 Las Palmas de Gran Canaria (ES)
(74) Representative: TRBL Intellectual Property

(57) **Abstract**

The invention refers to a method for selecting at least one bacterial species to be used as a bioinoculant for the promotion of growth of a marine phanerogam, wherein the method comprises: (a) isolating phosphate-solubilising bacteria from a sample of substrate obtained from the rhizosphere of a marine phanerogam, and (b) determining exopolysaccharide production, siderophore production and phosphatase activity of the bacterial species, wherein an exopolysaccharide production, a siderophore production, and a phosphatase activity above specific threshold values are indicative that the bacterial species can be used as a bioinoculant for the promotion of growth of a marine phanerogam. The invention also refers to a bioinoculant that comprises at least one bacterium from a species selected from the group consisting of *Methylobacterium phyllosphaerae, Methylobacterium sp., Microbacterium foliorum* and *Rhodococcus sp.* and related uses.

## Description

### FIELD OF THE INVENTION

The present invention refers to bioinoculant compositions made up of phosphate-solubilising bacteria (PSB), capable of promoting growth of marine phanerogams, and which can be used to assist and improve seagrass restoration and repopulation programs.

### BACKGROUND OF THE INVENTION

The monitoring of seagrass meadows made up of *Cymodocea nodosa* has warned of symptoms of regression of these ecosystems of great ecological importance in recent years. The urban development of coastal areas has led to the deterioration of some meadows and/or the disappearance of others. Concomitantly, a manifest alteration of the substrate where these plants are located is also evident. Marine phanerogam plants such as *Cymodocea nodosa* have specific nutrient requirements for their growth and development. Although conditions may vary depending on the species and the specific environment, the basic nutrient requirements are sunlight (to carry out photosynthesis), oxygen, and carbon dioxide (CO₂) as an essential carbon source for the synthesis of carbohydrates and other organic compounds. In addition, marine phanerogam plants require a suitable substrate to anchor, wherein the substrate provides inorganic nutrients, such as nitrogen (nitrate and ammonium), phosphorus, and trace elements (such as iron, manganese, and zinc), for its adequate growth.

Conservation strategies carried out *in-vitro* and *ex-vivo* for *Cymodocea nodosa* in the context of seagrass restoration and restocking programs have shown evidence of certain important requirements for the successful propagation and growth of this marine phanerogam plant:
1) replication through ramets requires a fragment of rhizome containing leaf and root, where phosphorus assimilation only takes place through roots buried in the soil; and
2) seed germination is conditioned by the existence of secondary dormancy, i.e., it develops when the seed has detached from the parent plant and requires environmental factors such as nutrient availability, mainly phosphorus.

From the above, it can be easily deduced that phosphorus is an essential nutrient for the development of ramets, allowing the *in-situ* viability of any fragment. Thus, phosphorus supplementation appears to be an important nutrient for the successful completion of conservation strategies of seagrass meadows.

In the case of terrestrial plants, it is widely known that phosphorus plays a role as a crucial element for plant development, since it is involved in vital processes such as photosynthesis, energy transfer, and the formation of compounds necessary for root development and reproduction. However, much of the phosphorus present in soil is in an insoluble form, making it inaccessible to plants. For this reason, the direct addition of phosphorus compounds in a form that plants can use contributes to the growth and maintenance of terrestrial plants.

More sustainable approaches have also shown that phosphate-solubilising bacteria (PSB) can play a critical role in soils by contributing to the bioavailability of phosphorus as one of the essential nutrients for plant growth. These strategies obviate the need of adding phosphorus directly as a fertiliser. These bacteria have the capability to release phosphorus from its inorganic insoluble form, such as tricalcium phosphate, aluminium phosphate, or ferric phosphate, turning it into a soluble form that plants can absorb. This process, known as phosphate solubilisation, involves the production of organic acids, or enzymes that break down inaccessible phosphate compounds, thereby releasing phosphorus in a form that plants can use for their growth and development. By increasing the availability of phosphorus for plants, these bacteria promote healthy plant growth and contribute to crop productivity. Since the use of PSB reduces supplementation with phosphorus as a fertiliser, these bacteria also minimise phosphorus runoff into water bodies, where it can cause pollution problems. Therefore, the ability of bacteria to release phosphorus from insoluble compounds is a prominent example of how microorganisms can play an essential role in nutrient dynamics in the biosphere, as they contribute to maintaining a sustainable phosphorus cycle in ecosystems. There are several species of bacteria that are known for their ability to solubilise phosphate in different types of soils and in various cultivars of terrestrial plants. In general, they belong to the genera of *Pseudomonas, Vibrio, Bacillus, Rhizobium, Enterobacter.* However, the effectiveness of these PSB may vary depending on specific soil conditions, including factors such as pH, insoluble phosphorus concentration, nutritional needs of cultivated plants, the presence of other microorganisms, and the excretion of substances and production of metabolites by bacteria.

In the case of marine plants, the prior art describes the association between certain microorganisms and the roots of marine plants. However, detailed description of function or synergies that may derive from this association is often lacking. In these regards, no studies appear to focus on the use of microorganisms, specifically PSB, for enhancing nutrient assimilation by marine plants. It should also be noted that, in aquatic ecosystems, fertilisers cannot be used as they are used in terrestrial ecosystems, since the risks of direct pollution are often a concern. Thus, a necessity exists in the art of the invention to provide strategies for the fertilisation of marine phanerogam plans that could assist and improve seagrass restoration and repopulation programs.

### DETAILED DESCRIPTION OF THE INVENTION

In view of the above needs in the instant art, the inventors have found that specific phosphate-solubilising bacteria (PSB) which may be isolated from the vicinity of roots of certain marine plants - and which may be selected based on their properties in regards exopolysaccharide production, siderophore production, and phosphatase activity - can be used as fertilisers to promote the growth of phanerogams such as of *Ruppia maritima, Cymodocea nodosa,* and *Posidonia oceanica.*

Thus, in a first aspect the invention relates to a method for selecting at least one bacterial species to be used as a bioinoculant for the promotion of growth of a marine phanerogam, wherein the method comprises:
a) isolating bacterial species from a sample of substrate obtained from the rhizosphere of a marine phanerogam, wherein the bacterial species are capable of growing in a medium with a source of inorganic phosphorus,
b) determining exopolysaccharide production, siderophore production and phosphatase activity of the bacterial species
wherein:
- an exopolysaccharide production in excess of 200 mg/L;
- a siderophore production in excess of 2% by weight; and
- a phosphatase activity in excess of 7.5 e-3 M p-nitrophenol
are indicative that the bacterial species can be used as a bioinoculant for the promotion of growth of a marine phanerogam.

In the context of the instant invention, the term "bioinoculant" (also known as microbial inoculant or soil inoculants) refers to an environmental solution based on supplying beneficial rhizosphericic or endophytic microorganisms to the substrate of plants in order to promote health, growth and/or development. Specifically, the invention describes bioinoculants that comprise phosphate-solubilising bacteria (PSB), capable of transforming inorganic soil phosphates to forms that enable uptake by plants. Based on these properties, bioinoculants can be used as fertilisers. In particular applications, PSB can substitute the need for supplementation with external phosphate fertilisers. Since PSB can transform inorganic phosphates into bioavailable forms, they are naturally capable of growing in a medium with a source of inorganic phosphorus. This property may be used to isolate and select bacteria within the context of the invention.

In addition to their capacity to grow in a medium with a source of inorganic phosphorus, the bacterial species of the instant invention are also further selected based on three criteria: exopolysaccharide (EPS) production, siderophore production and phosphatase activity.

Exopolysaccharides (EPS) are long-chain polysaccharides comprising repeating units of sugars, mainly glucose, galactose and rhamnose, in different ratios. EPS are natural products excreted by certain genera of bacteria, which contribute to loosening the substrate (decompaction), favouring gas exchange and root growth (Mingxing Qi et al., "Exopolysaccharides from Marine Microbes: Source, Structure and Application", Mar Drugs. 2022 Aug; 20(8): 512). In these regards, the inventors' preliminary studies have deemed relevant that, in addition to the ability to release phosphate, bacteria also contribute to the decompaction of the substrate where it is anchored, to favour the exchange of gases (oxygen, for example) and thus, favour the growth of roots. The bacterial species selected by the method of the present invention have an exopolysaccharide production in excess of 200 mg/L. In particular embodiments, the bacterial species selected by the method of the present invention have an exopolysaccharide production in excess of 200 mg/L, 300 mg/L, 400 mg/L, or 500 mg/L. In particular embodiments, exopolysaccharide production is within a range of 200-700 mg/L; within a range of 300-600 mg/L; or within a range of 400-500 mg/L.

Siderophores are low molecular weight, strong iron-binding molecules produced by microbes. These molecules assist in the absorption of iron. For this reason, they are excreted by bacteria to retain iron around them (Jianwei Chen et al., "Chemistry and Biology of Siderophores from Marine Microbes", Mar Drugs. 2019 Oct; 17(10): 562). The bacterial species selected by the method of the present invention have a siderophore production in excess of 2%. In the context of the invention, siderophore production is analysed using the method of Schwyn and Neilands ("Universal Chemical Assay for the Detection and Determination of Siderophores‴, Analytical Biochemistry 160,47-56, 1987). Under this determination method, the calculation of siderophore production is carried out based on the absorbance of an iron-comprising CAS complex, which includes iron (Fe) and a fluorophore. The CAS solution is then added into a solution with siderophores, and the siderophores sequester iron from the CAS complex (that is, they "steal the iron" from the CAS complex). The change in colour of the fluorophore in the iron-comprising complex (CAS complex) before and after the siderophore has sequestered iron is expressed in percentage values (%). The result thus obtained (value expressed in %) indicates the Fe that has been sequestered by the siderophores. By way of an example, if the percentage value is 10%, this is to be interpreted as meaning that 10% of the iron in the medium was sequestered by the siderophores produced by the bacterium compared to a control (100% value in the control). In particular embodiments, the bacterial species selected by the method of the present invention have a siderophore production in excess of 2%, 4%, 6%, 8%, 10%, 12%, 14%, or 16%. In particular embodiments, siderophore production is within a range between 2-16%, within a range between 4-14%, within a range between 6-12%, or within a range between 8-10%.

Extracellular phosphatases are a significant component of most marine algae and bacteria and play a prominent role in the recycling of organic P and in the avoidance of P limitation in the sea (Hoppe, H.-G., "Phosphatase activity in the sea", Hydrobiologia 493: 187-200, 2003.). *A priori,* all PSB have this enzymatic activity. In the context of the instant invention, bacteria with a relatively high phosphatase activity have been selected. The bacterial species selected by the method of the present invention have a phosphatase activity in excess of 7.5 e-3 M p-nitrophenol. In particular embodiments, the bacterial species selected by the method of the present invention have a phosphatase activity in excess of 7.5 e-3 M p-nitrophenol, 1.0 e-2 M p-nitrophenol, 1.5 e-2 M p-nitrophenol, or 2.0 e-2 M p-nitrophenol. In particular embodiments, phosphatase activity is within a range of 7.5 e-3 to 2.5 e-2 M p-nitrophenol, or within a range of 1.0 e-2 to 2.0 e-2 M p-nitrophenol.

In a particular embodiment of the present invention, exopolysaccharide production is within a range of 200-700 mg/L; siderophore production is within a range between 2-16% by weight; and phosphatase activity is within a range of 7.5 e-3 to 2.5 e-2 M p-nitrophenol.

As defined herein, the bacteria of the invention are obtained from the rhizosphere of the marine phanerogam plant. The term "rhizosphere" refers to the narrow region of soil or substrate in the immediate vicinity of the marine plant that is directly influenced by root secretions and associated soil microorganisms (also known as the root microbiome). In particular embodiments of the invention, the rhizosphere is within 1 mm-10mm from the roots of the marine phanerogam plant. In particular embodiments of the invention, the rhizosphere is within 10mm, within 9mm, within 8mm, within 7mm, within 6mm, within 5mm, within 4mm, within 3mm, within 2mm, or within 1mm from the roots of the marine phanerogam plant.

In the context of the invention, the term "promotion of growth" refers generally to the physiological health, growth and/or development of a marine phanerogam plant. The term "health" describes lack of evident signs of plant injury or disease. The term "growth" refers to the process of increasing in plant size. The term "development" defines the formation of the correct plant structures. In the context of the invention, and since the growth of marine phanerogams is very slow (estimated at 0.1 cm year⁻¹), the promotion of growth of a marine phanerogam plant can be monitored by means of measuring the fluorescence of chlorophyll. In particular, the maximum efficiency of chlorophyll is measured by the Fv/Fm parameter, and this measurement is indicative of how efficiently chlorophyll captures light and transforms light into chemical energy.

In a particular embodiment of the invention, the physiological state of the marine plant is measured according to the maximum photochemical quantum yield of Photosystem II (PSII), and thus, the promotion of growth of a marine phanerogam is monitored by measurement of fluorescence parameters F₀ and Fᵥ/F_{M}, wherein F₀ corresponds to the initial fluorescence, Fv corresponds to the variable fluorescence and F_{M} corresponds to the maximum fluorescence (Lichtenthaler et al., "How to correctly determine the different chlorophyll fluorescence parameters and the chlorophyll fluorescence decrease ratio RFd of leaves with the PAM fluorometer", Photosynthetica 43 (3): 379-393, 2005). It is within the ability of the person skilled in the art of the present invention to select the most appropriate fluorometer type for the determination of the above fluorescence parameters F₀ and Fᵥ/F_{M}. For example, Walz Photosynthesis Instruments' JUNIOR-PAM and the like may be used in order to work the instant invention.

In the context of the invention, the term "phanerogam" (also known as a seed plant or a spermatophyte) refers to plants that produce seeds. More specifically, marine phanerogams are a group of plants within the category of vascular flowering, fruit-bearing plants with seeds, which grow under water. Marine phanerogams develop into 'seagrass meadows', which are groups of plants that belong to the same species and that are connected among them. The diversity of marine phanerogam plants is higher on the coast and on continental platforms and lower in deeper sea. Significant species of marine phanerogams are *Posidonia oceanica, Cymodocea nodosa, Ruppia maritima* and *Zostera noltii.* Thus, in particular embodiments of the invention, the marine phanerogam is selected from the group consisting of *Ruppia maritima, Cymodocea nodosa, Posidonia oceanica,* and combinations thereof.

In another aspect, the invention refers to the use of at least one bacterium from a genus selected from the group consisting of *Methylobacterium, Microbacterium,* and *Rhodococcus* as a bioinoculant for the promotion of growth of a marine phanerogam. The term *"Methylobacterium"* refers to a genus of bacteria in the order Hyphomicrobiales and the family Methylobacteriaceae. In a particular embodiment, the bacterium of the genus *Methylobacterium* is a bacterium of the species *Methylobacterium phyllosphaerae* or of the species *Methylobacterium* sp. The term *"Microbacterium"* refers to a genus of bacteria in the family Microbacteriaceae. In a particular embodiment, the bacterium of the genus *Microbacterium* is a bacterium of the species *Microbacterium foliorum.* The term *"Rhodococcus"* refers to a genus of bacteria in the family Nocardiaceae. In a particular embodiment, the bacterium of the genus *Rhodococcus* is a bacterium of the species *Rhodococcus sp.* In a particular embodiment, the invention refers to the use of at least one bacterium from a species selected from the group consisting of *Methylobacterium phyllosphaerae, Methylobacterium* sp., *Microbacterium foliorum* and *Rhodococcus sp.* as a bioinoculant for the promotion of growth of a marine phanerogam.

The bacterial genera or species of the invention may be used individually, or they may be used in different combinations forming a bacterial consortium.

Thus, when used individually in particular embodiments of the invention, the bioinoculant comprises, as the only active bacterial species, *Methylobacterium phyllosphaerae,* or *Methylobacterium sp.,* or *Microbacterium foliorum,* or *Rhodococcus sp.* Alternatively, when used as part of a bacterial consortium in particular embodiments of the invention, the bioinoculant comprises, as the only active bacterial species, *Methylobacterium phyllosphaerae* and *Methylobacterium sp.,* or *Methylobacterium phyllosphaerae* and *Microbacterium foliorum,* or *Methylobacterium phyllosphaerae* and *Rhodococcus sp.,* or *Methylobacterium sp.,* and *Microbacterium foliorum,* or *Methylobacterium sp.* and *Rhodococcus sp.,* or *Microbacterium foliorum* and *Rhodococcus sp.,* or *Methylobacterium phyllosphaerae, Methylobacterium sp.* and *Microbacterium foliorum,* or *Methylobacterium phyllosphaerae, Methylobacterium sp.* and *Rhodococcus sp.,* or *Methylobacterium phyllosphaerae, Microbacterium foliorum* and *Rhodococcus sp.,* or *Methylobacterium sp., Microbacterium foliorum* and *Rhodococcus sp.,* or *Methylobacterium phyllosphaerae, Methylobacterium sp., Microbacterium foliorum* and *Rhodococcus sp.* In particular embodiments, the bioinoculant is a bacterial consortium that comprises at least 70% of bacteria of the genus *Methylobacterium.*

In a further aspect, the present invention refers to a bioinoculant for the promotion of growth of a marine phanerogam, wherein the bioinoculant comprises at least one bacterium from a genus selected from the group consisting of *Methylobacterium, Microbacterium,* and *Rhodococcus.* In particular embodiments, the bioinoculant comprises at least one bacterium from a species selected from the group consisting of *Methylobacterium phyllosphaerae, Methylobacterium sp., Microbacterium foliorum* and *Rhodococcus sp.* In a particular embodiment, the bioinoculant is a bacterial consortium comprising all of *Methylobacterium phyllosphaerae, Methylobacterium sp., Microbacterium foliorum,* and *Rhodococcus sp.* In further embodiments, the bioinoculant comprises at least one bacterial strain selected from the group consisting of *Methylobacterium phyllosphaerae* [Deposit No CECT 31071.], *Rhodococcus sp.* [Deposit No. CECT 31072], *Methylobacterium* sp. [Deposit No. CECT 31073] and *Microbacterium foliorum* [Deposit No. CECT 31074]. The strains have been deposited in the Spanish Collection of Type Cultures (CECT), Building 3 CUE, University of Valencia Science Park, c/ Catedrático Agustin Escardino, 9, 46980 Paterna (Valencia), Spain.

The bacteria of the invention may be cultivated in a suitable medium for the preparation of the bioinoculant. Suitable media include, but are not limited to:
- marine agar media (Medium F, which contained Yeast Extract 0.5 g/L, Dextrose 10 g/L, Ammonium sulphate 0.5 g/L, Potassium chloride 0.2 g/L, Magnesium sulphate 0.1 g/L, Manganese sulphate 10⁻⁴ g/L, Iron sulphate 10⁻⁴ g/L, Agar 15 g/L), wherein the marine agar medium is supplemented with at least one of the following phosphate salts (Ca₃(PO₄)₂, FePO₄ or AlPO₄) at a concentration of 5 g/L.
- Marino Agar (Ammonium nitrate 0,0016 g/L, Bacteriological agar 15 g/L, Bacteriological peptone 5 g/L, Boric acid 0,022 g/L, Calcium chloride 1,8 g/L, Disodium phosphate 0,008 g/L, Magnesium chloride anhydrous 8,8 g/L, Potassium bromide 0,08 g/L, Potassium chloride 0,55 g/L, Sodium bicarbonate 0,16 g/L, Sodium chloride 19,4 g/L, Sodium fluoride 0,0024 g/L, Sodium silicate 0,004 g/L, Sodium sulphate 3,24 g/L, Strontium chloride 0,034 g/L, Yeast extract 1 g/L, Ferric citrate 0,1 g/L).
- R2A Agar (Yeast Extract 0.5 g/l, Proteose Peptone 0.5 g/l, Casein Hydrolysate 0.5 g/l, Glucose 0.5 g/l, Starch 0.5 g/l, K2HPO₄ 0.3 g/l, MgSO4 0.024 g /l, Sodium Pyruvate 0.3 g/l, Agar 15 g/l).

In another aspect, the invention relates to a starter substrate, wherein the starter substrate comprises 0.5-1.0 ml of a bacterial culture at 10⁸ cells/ml of a bioinoculant according to the instant invention, per milligram of substrate. Examples of substrates that may be used in the context of the instant invention include 3DM reef-sand, 3MM fine coral sand, 6MM fine coral sand, and the like. In the context of the invention, the term "starter substrate" refers to a primary soil preparation that is initially used to seed the bioinoculant of the invention so that the bacteria of the invention may become acclimatised, prior to starting any marine plant cultivation. That is, the starter substrate is not used to seed marine plants into it.

In a further aspect, the invention relates to a dosing substrate, wherein the dosing substrate comprises a 1:1 weight mixture of a mesocosm substrate and a starter substrate according to the instant invention. In the context of the invention, the term "dosing substrate" refers to a secondary soil preparation that is used to seed marine plants.

In a final aspect, the invention relates to the use of a bioinoculant according to the invention, to the use of a starter substrate according to the invention, or to the use of a dosing substrate according to the invention, to promote growth of a marine phanerogam.

All the terms and embodiments described herein are equally applicable to all aspects of the invention. It should be noted that, as used in the specification and in the appended claims, the singular forms "a", "an", and "the" include their plural referents unless the context clearly indicates otherwise. Similarly, the term "comprises" or "comprising" as used herein also describes "consists of" or "consisting of" in accordance with generally accepted patent practice.

### EXAMPLES

The following invention is hereby described by way of the following examples, which are to be construed as merely illustrative and not limitative of the scope of the invention.

### Example 1: Design of a suitable substrate for marine phanerogams

The inventors' previous *in vitro* (laboratory) and ex *vivo* (mesocosm) experimental work has demonstrated the need for phosphorus availability for the correct development of marine plants. Taking a comprehensive view of the plant and its environment *(in situ),* the inventors have carried out preliminary studies to identify phosphate solubilising bacteria in the different areas where the *Ruppia maritima* and *Cymodocea nodosa* plants grow and flourish. The soils surrounding these two plants in investigated areas are structurally different in terms of sediment composition, but also in terms of promoting the growth of phanerogams when used in mesocosms (large aquariums). Microbial assessments of the environments surrounding these two plants showed differences between the bacterial populations. Different bacterial types were also found in the surroundings of the rhizospheres of the two species. In view of these results, the inventors decided to identify, characterise, and select bacterial species in order to create a consortium of microorganisms that could be used as a bioinoculant for the correct development of marine phanerogams.

### Example 2: Characterisation of bacterial species in the surroundings of rhizospheres

Soil surrounding the roots of *Cymodocea nodosa* was extracted from a seagrass meadow in Juan Grande (27°48'00"N; 15°25'40"W), Gran Canaria. A 100 g sample of such substrate was selected, and this substrate was resuspended in sterile seawater (200 ml) for 30 min. From this aqueous suspension, 0.5 ml aliquots were taken for plate culture of bacterial species present in the sample. Bacteria were grown in a marine agar media (Medium F, which contained Yeast Extract 0.5 g/L, Dextrose 10 g/L, Ammonium sulphate 0.5 g/L, Potassium chloride 0.2 g/L, Magnesium sulphate 0.1 g/L, Manganese sulphate 10⁻⁴ g/L, Iron sulphate 10⁻⁴ g/L, Agar 15 g/L) and phosphate was added to the marine agar medium in different complex forms. That is, the medium was supplemented with at least one of the following phosphate salts (Ca₃(PO₄)₂, FePO₄ or AlPO₄) at a concentration of 5 g/L.

Bacterial strains capable of growing in the above medium (i.e., a medium with a source of inorganic phosphorus) were selected. Pure bacterial colonies were obtained from these media after successive replications. All pure colonies were kept at low temperatures to maintain colony traceability. Samples were conserved with glycerol 20% at -20°C in order to create a bank of bacterial strains. Each of the colonies grown in the above media was subjected to molecular identification by means of DNA extraction, amplification of specific areas, purification, etc. PCR amplification segments of specific areas of the DNA of each bacterium were studied. Universal or domain-specific primers were used (see sequences in Liu et al 1997, App. Env. Microbiol. 63:11). This task is complemented by the design of phylogenetic trees for the precise molecular identification of the strains thus obtained, compared to those deposited in the databases.

In total, 16 species of phosphate-solubilising bacteria were isolated and molecularly identified by means of 16s RNA markers. The 16 originally identified species were: (1) *Bacillus halotolerans subtilis mojavensis,* (2) *Bacillus licheniformes haynesii,* (3) *Bacillus safensis pumilus,* (4) *Brachybacterium paraconglometarum,* (5) *Brevundimonas staleyi, (6) Methylobacterium fujisawaense,* (7) *Methylobacterium phyllosphaerae, (8) Methylobacterium sp.,* (9) *Methylorubrum sp.,* (10) *Microbacterium foliorum,* (11) *Microbacterium phyllosphaerae,* (12) *Myceligenerans xiligouense,* (13) *Priestia megaterium aryabhattai,* (14) *Rhodococcus sp.,* (15) *Rhodotorula mucilaginosa,* (16) *Roseomonas aestuarri.*

### Example 3: Selection of bacterial species for a growth-promoting bioinoculant

Once the bacterial species where properly characterised and identified, they were further selected based on three criteria:
- Exopolysaccharide production. Exopolysaccharides (EPS) are natural products excreted by certain genera of bacteria. EPS contribute to loosening the substrate (decompaction), favouring gas exchange and root growth.
- Siderophore production. Siderophores are compounds that assist in the absorption of iron, and which are excreted by bacteria to retain iron around them. Iron and phosphorus are related to each other because they are both essential for plants and form the most abundant insoluble complexes in the form of iron phosphate (FePO₄). In this context, it is important that iron is not solubilised and lost when the iron phosphate complex breaks. For this reason, it is also important that the phosphate solubilising bacteria (PSB) have the capacity to produce siderophores so that there is no deficit of any nutrient, particularly iron, during growth of the marine plant.
- Phosphatase activity. *A priori,* all PSB have this enzymatic activity, but only the most active bacteria were selected in the context of the instant invention.

The selection of the bioinoculant was based on a factor quality analysis method that allows correlating the measurements of exopolysaccharide production, siderophore production and phosphatase activity of the phosphate solubilising strains:
*1. Production of exopolysaccharide:* Bacterial strains capable of growing in an inorganic phosphate-rich media (non-agar F medium) were cultured, under stress conditions, in the same medium until visible EPS production was determined as previously described (Dubois, M., Gilles, K. A., Hamilton, J. K., Rebers, P. A., & Smith, F. A. J. N. (1951). A colorimetric method for the determination of sugars. Nature, 168(4265), 167-167). EPS was precipitated at a 1:3 ratio of culture medium: ethanol and kept at 4° C overnight. EPS was then centrifuged at 9000 rpm for 10 min. and the pellet was used for EPS titration. To do this, 0.014 ml of 80% phenol and 1.42 ml of H₂SO₄ was added to 0.6 ml of resuspended pellet. Samples were left to stabilise for 10 min and then they were stirred and placed in a water bath at 30° C for 20 min. Absorbance was measured at 490 nm. The calibration curve was obtained with known glucose concentrations.
*2. Siderophore production:* Siderophore production was analysed using the method of Schwyn and Neilands ("Universal Chemical Assay for the Detection and Determination of Siderophores‴, Analytical Biochemistry 160,47-56, 1987). Bacteria were inoculated in an iron-deficient medium (K₂HPO₄, 0.1 g/L; KH₂PO₄, 3.0 g/L; MgSO₄ 7H₂O, 0.2 g/L; (NH₄)2SO₄, 1.0 g/L; succinic acid, 4.0 g/L) at 30° C in a stirrer at 130 rpm for 48 h. The supernatant was analysed for siderophore production using the Universal Chrome Azurol-S (CAS) colorimetric assay. The absorbance was read at 630 nm for loss of blue colour. The activity was recorded as a percentage of siderophores: [(Aᵣ-Aₛ) x (Aᵣ-1) x 100], where Aᵣ is the Azurol of the uninoculated medium + CAS solution, and where Aₛ is the Azurol of the supernatant (culture supernatant + CAS solution).
*3. Determination of phosphatase activity*: The determination of active colonies in phosphatase production was assessed according to Nahas, E., et al. ("Microrganismos solubilizadores de fosfato e produtores de fosfatases de vários solos", Revista Brasileira de Ciência do Solo, 18(1), 43-48, 1994). For the enzymatic assay, pure colonies were independently weighed and re-suspended in a universal buffer (0.17 M) adjusted to pH 5.5 and 9.0 for the determination of acid and alkaline phosphatases, respectively. 1 ml of p-nitrophenylphosphate (0.025 M) was added to 3 ml of the bacterial suspension. The reaction mixtures were incubated for 3 hours at 37°C and centrifuged at 2000 g for 10 minutes. 4.5 mL of NaOH (0.5 M) was added to 0.5 mL of the supernatant. The amount of released p-nitrophenol was determined by measuring the absorbance of the solution at 410 nm. The results of enzymatic activities were expressed in mg of p-nitrophenol by weight colony at 37°C. To assess phosphatase activities in the soil directly, and to be able to extract p-nitrophenol from the soil, 1 ml of CaCl₂ (0.5M) and 4 ml of NaOH (0.5M) were used, respectively (Burns 1978). The mixtures were centrifuged at 3000 g for 10 min and filtered through a No. 2 Whatman paper. The formation of p-nitrophenol was also determined by spectrophotometry at 410 nm.

The bacteria were selected according to the following selection criteria:
- exopolysaccharide production in excess of 200 mg/L;
- siderophore production in excess of 2% by weight; and
- phosphatase activity in excess of 7.89 e-3 M p-nitrophenol

Following the analysis of the selection criteria, the following four (4) species were selected to establish the bacterial consortium out of the 16 species that had been originally identified: (1) *Methylobacterium sp.,* (2) *Methylobacterium phyllosphaerae, (3) Microbacterium foliorum* and (4) *Rhodococcus* sp.

These bacteria showed that following characteristics:
- EPS producers in a range of 202.58-655.01 mg/L;
- siderophore producers in a range between 2.27-15.78% by weight; and
- phosphatase activity in a range of 7,89 e-3 ± 8,32 e-4 to 2,12 e-2 ± 2,07 e-3 M p-nitrophenol.

The specific values corresponding to the identified species are indicated in table 1.

**Table 1: Specific values for EPS production, phosphatase activity, and siderophore production, corresponding to the identified species.**

| **SPECIES** | **EPS (mgL-1)** | **Siderophores (%)** | **Phosphatases (M p-nitrophenol)** |
|---|---|---|---|
| *Rhodococcus sp.* | 356,92 | 15,37 | 1,75E-02 |
| *Methylobacterium sp.* | 398,04 | 2,27 | 8,07E-03 |
| *Microbacterium foliorum* | 202,58 | ND | 1,95E-02 |
| *Methylobacterium phyllosphaerae* | 360,28 | 3,13 | 1,68E-02 |

The remaining species initially selected did not meet at least two of the established requirements, i.e., EPS production, phosphatase activity, and siderophore production.

### Example 4: Preparation of inoculated substrate

Next, the inventors identified (1) optimal substrate conditions for efficient growth of the bacterial inoculum and (2) optimal substrate conditions for efficient growth of marine plants when inoculated with the selected substrate.

Optimisation of the inoculated substrate (for inoculum growth): Selected bacterial strains were cultured in F medium to a concentration of 10⁸ cells/ml. The substrate (3DM reef sand, 3MM fine coral sand, 6MM fine coral sand, African sand) was inoculated with each of the selected species. A volume in millilitres (ml) of the bacterial culture at this 10⁸ cells/ml concentration was added, respectively:
(A) RATIO 1:1, to the same volume but in weight (g) of substrate, wherein 1 culture volume (10⁸ cells/ml) was added to 1 volume by weight (g) of substrate, that is, a ratio of 20 ml bacterial culture at 10⁸ cells/ml: 20 mg substrate,
(B) RATIO 1:2, to twice the weight (g) of that volume, wherein 1 culture volume (10⁸ cells/ml) was added to 2 volumes by weight (g) of substrate, that is, a ratio of 20 ml bacterial culture at 10⁸ ml⁻¹ cells: 40 mg substrate, and
(C) RATIO 1:3, to three times the weight (g) of that volume, wherein 1 culture volume (10⁸ cells/ml) was added to 3 volumes by weight (g) of substrate, that is, a ratio of 20 ml bacterial culture at 10⁸ ml⁻¹ cells: 60 mg substrate,
and maintained for 5 days.

After 5 days, the concentration of surviving bacteria was measured. The best growth was obtained for the ratios (A) 1:1 and (B) 1:2. These ratios were selected as inoculated substrate, or starter substrate.

Optimisation of the dosing substrate (for plant growth): A portion (100 gr) of inoculated substrate was added to an equal part of the substrate (3DM reef sand, 3MM fine coral sand, 6MM fine coral sand, African sand) of an aquarium (mesocosm) at a 1:1 ratio (100g inoculated substrate to 100g mesocosm), at a 1:2 ratio (100g inoculated substrate to 200g mesocosm) and at a 1:3 ratio (100g inoculated substrate to 300 gr mesocosm). These mixtures were left to rest (acclimatise) for another 5 days to measure the concentration of bacteria. The best results in regards dosage substrate were obtained with 100g of inoculated substrate A to 100g of mesocosm (1:1 ratio); and 100g substrate A to 2 inoculated substrate (200 g mesocosm) (ratio 1:2).

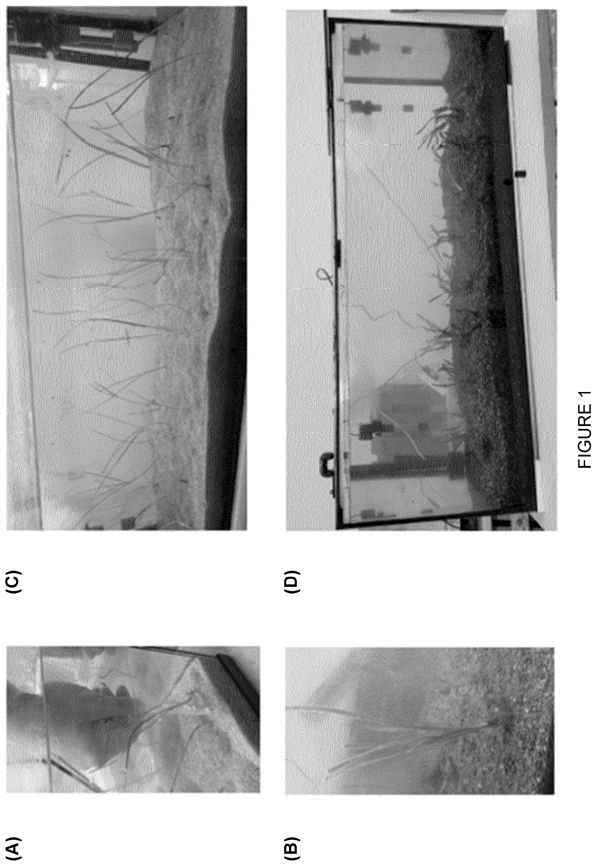

Once the two optimal mesocosm mixtures were acclimatised, fragments of *Cymodocea nodosa* were planted, wherein the fragments contained rhizome, root, leaf (figure 1). The physiological state of the marine plants was measured according to the maximum photochemical quantum yield of Photosystem II (PSII) in each of the mesocosm whose parameters were F₀ and Fᵥ/F_{M}.

The physiological state of marine plants, at 15 days, improved considerably in those mesocosms with a 1:1 ratio of 100g of inoculated substrate A to 100 g of aquarium mesocosm, wherein the inoculated substrate A was made up of 20 ml of bacterial culture at 10⁸ cells/ml to 20 mg substrate. In this culture system, the parameters indicating the physiological state of the marine plants were F₀: 423-556 and Fᵥ/F_{M}: 0.49-0.606. Comprehensive results are shown in table 1 herein below.

**Table 1: Parameters indicating the physiological state of the marine plants for different types of dosing substrate tested.**

| Inoculated Substrate | Dosing Substrate | |
|---|---|---|
| | **Ratio 1:1** | **Ratio 1:2** |
| | (Inoculated Substrate to Mesocosm Substrate) | (Inoculated Substrate to Mesocosm Substrate) |
| **Substrate A:** | F₀: 423-556 | Non-viable C. *nodosa* fragment at 15 d |
| 20 ml bacterial culture at 10⁸ cells ml⁻¹ to 20 mg substrate | | |
| | Fᵥ/F_{M}: 0.49-0.606 | |
| **Substrate B:** | F₀: 191-247 | Non-viable C. *nodosa* fragment at 15 d |
| 20 ml bacterial culture at 10⁸ cells ml⁻¹ to 40 mg substrate | | |
| | Fᵥ/F_{M}:0.760-774 | |

### BRIEF DESCRIPTION OF THE FIGURES

**Figure 1****:** Development and transplantation of *Cymodocea nodosa:* individual plant (A, B) and culture overview (C); and of *Posidonia oceanica:* culture overview (D).

## Claims

1. A method for selecting at least one bacterial species to be used as a bioinoculant for the promotion of growth of a marine phanerogam, wherein the method comprises:
a) isolating bacterial species from a sample of substrate obtained from the rhizosphere of a marine phanerogam, wherein the bacterial species are capable of growing in a medium with a source of inorganic phosphorus,
b) determining exopolysaccharide production, siderophore production and phosphatase activity of the bacterial species
wherein:
- an exopolysaccharide production in excess of 200 mg/L;
- a siderophore production in excess of 2% by weight; and
- a phosphatase activity in excess of 7.5 e-3 M p-nitrophenol
are indicative that the bacterial species can be used as a bioinoculant for the promotion of growth of a marine phanerogam.

2. The method according to claim 1, wherein:
- exopolysaccharide production is within a range of 200-700 mg/L;
- siderophore production is within a range between 2-16% by weight; and
- phosphatase activity is within a range of 7.5 e-3 to 2.5 e-2 M p-nitrophenol.

3. Use of at least one bacterium from a genus selected from the group consisting of *Methylobacterium, Microbacterium,* and *Rhodococcus* as a bioinoculant for the promotion of growth of a marine phanerogam.

4. The use according to claim 3, wherein the at least one bacterium is from a species selected from the group consisting of *Methylobacterium phyllosphaerae, Methylobacterium sp., Microbacterium foliorum* and *Rhodococcus sp.*

5. A bioinoculant for the promotion of growth of a marine phanerogam, wherein the bioinoculant comprises at least one bacterium from a genus selected from the group consisting of *Methylobacterium, Microbacterium,* and *Rhodococcus.*

6. The bioinoculant according to claim 5, wherein the bioinoculant comprises at least one bacterium from a species selected from the group consisting of *Methylobacterium phyllosphaerae, Methylobacterium sp., Microbacterium foliorum* and *Rhodococcus sp.*

7. The bioinoculant according to any one of claims 5 or 6, wherein the bioinoculant is a bacterial consortium comprising *Methylobacterium phyllosphaerae, Methylobacterium sp., Rhodococcus sp.,* and *Microbacterium foliorum.*

8. The bioinoculant according to any one of claims 5 to 7, wherein the bioinoculant comprises at least one bacterial strain selected from the group consisting of *Methylobacterium phyllosphaerae* [Deposit No. CECT 31071], *Rhodococcus sp.* [Deposit No. CECT 31072], *Methylobacterium sp.* [Deposit No. CECT 31073] and *Microbacterium foliorum* [Deposit No. CECT 31074].

9. A starter substrate, wherein the starter substrate comprises 0.5-1.0 ml of a bacterial culture at 10⁸ cells/ml of a bioinoculant according to any one of claims 5 to 8 per milligram of substrate.

10. A dosing substrate, wherein the dosing substrate comprises a 1:1 weight mixture of a mesocosm substrate and a starter substrate according to claim 9.

11. Use of a bioinoculant according to any one of claims 5 to 8, of a starter substrate according to claim 9, or of a dosing substrate according to claim 10, to promote growth of a marine phanerogam.

12. The method according to any one of claims 1 or 2, the use according to any one of claims 3 or 4, the bioinoculant according to any one of claims 5 to 8, the starter substrate according to claim 9, or the dosing substrate according to claim 10, wherein the marine phanerogam is selected from the group consisting of *Ruppia maritima, Cymodocea nodosa, Posidonia oceanica,* and combinations thereof.
